# EUROPEAN PATENT APPLICATION

(11) **EP 0 538 844 A2**
(43) Date of publication of application: **28.04.1993**
(21) Application number: 92118033.7
(22) Date of filing: 21.10.1992
(51) Int. Cl.: C07D 455/03, A61K 31/435

(54) **Tetrahydroprotoberberine quaternary ammonium compounds and the process for preparing thereof**

(30) Priority: 21.10.1991 CN 91107511
(71) Applicant: CHINA PHARMACEUTICAL UNIVERSITY, Nanjing (CN); ADMINISTRATIVE CENTER OF NEW DRUG RESEARCH, THE STATE PHARMACEUTICAL ADMINISTRATION OF CHINA, Beijing (CN)
(72) Inventor: Peng, Sixun, Nanjing (CN); Dai, Dezai, Nanjing (CN); Huang, Zhenya, Nanjing (CN); Huang, Wenglong, Nanjing (CN); Wang, Youqun, Nanjing (CN); Zhang, Can, Nanjing (CN); Yu, Feng, Nanjing (CN)
(74) Representative: von Füner, Alexander, Dr.

(57) **Abstract**

A tetrahydroprotoberberine quaternary ammonium compound represented by the general formula:

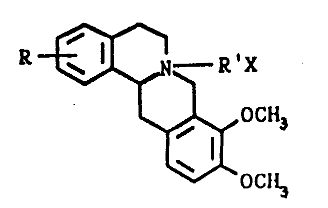

R,R',X are as defined in the specification. Protoberberine is reduced by potassium borohydride to tetrahydroprotoberberine (I). Tetrahydroprotoberberine quaternary ammonium compounds are prepared by reaction of tetrahydroprotoberberine with halohydrocarbon in acetone or ethanol. These compounds can suppress arrhythmia and ventricular fibrillation, protect myocardial is chemia, and can be conveniently orally administered.

## Description

The invention relates to tetrahydroprotoberberine quaternary ammonium compounds and their preparation process.

Cardiovascular disease is one of the main diseases seriously threatening human health. Arrhythmia is a common cardiovascular disease. At present, the antiarrhythmic agents used clinically, such as Quinidine, Lidocaine, Flecainide, Encainide, Propafenone, Propranolol and Anmiodarone, have cvrative effects upon arrhythmia,but each has variovs drawbacks. For example, Flecainide, used to prolong the action potential period, has a good effect upon arrhythmia, but is limited in clinical use, especially in the prevention of sudden death caused by ventricular fibrillation, and by the marked side effects of pulmonary fibrosis and thyroid function change. Another agent, Lidocaine, can effectively suppress arrhythmia, but can not effectively prevent ventricular fibrillation, and can not be administered orally.

Novel antiarrhythmic drugs are being developed in order to overcome the defects of the present antiarrhythmic drugs. Fukuda H. et al reported that protoberberine, a widely spread alkaloid, possessed a variety of bioactivites. In particular, berberine, palmatine and berberubine showed highly potent cardiovascular activities. The purpose of this invention is to study novel antiarrhythmic drugs with low side-effects and convenient administration,based upon active chinese herbal medicinal components, such as berberine.

The inventor has found that a now compound, 7-(4-chlorobenzyl)-5,8,13,13a-tetrahydroberberine chloride, obtained by structural modification of protoberberine, possesses potent antiarrhythmic properties. This invention discloses a series of compounds shown by the following general formula: 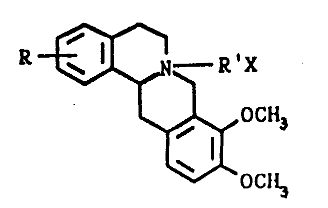
Wherein

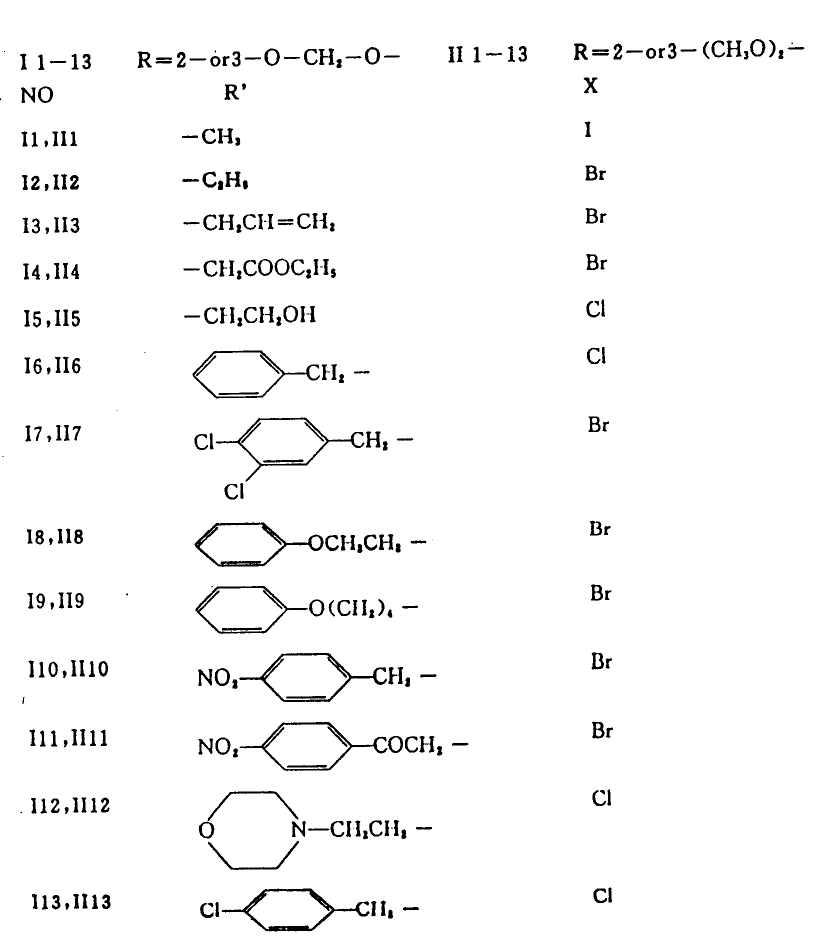

This invention discloses the process for preparing the above tetrahydroprotoberberine quaternary ammonium compound, which comprises two steps. First, protoberberine is reduced by potassium borohydride to tetrahydroprotoberberine. Second, tetrahydroprotoberberine reacts with a suitable halohydrocarbon to produce tetrahydroprotoberberine quaternary ammonium compounds. In the second step, another method has been reported, but with a relatively long reaction time and low yield, (Tanka, Satoru; Ueda, Koichiro. Jpn Kokai Tokyo Koho. 78,130,697). The method disclosed in the instant invention is characterized by producing high yields and pure products, and comprises three means to prepare different compounds:
(A)reaction of tetrahydroprotoberberine with excess halohydrocarbon at 100-110°C for 1.5-10 hours;
(B)heating tetrahydroprotoberberine and halohydrocarbon (aromatic halide substituted by nitro group) in acetone under reflux; and
(C)heating tetrahydroprotoberberine and halohydrocarbon with heterocycle in ethanol under reflux.

The preparation process of the invention is described by the following scheme: 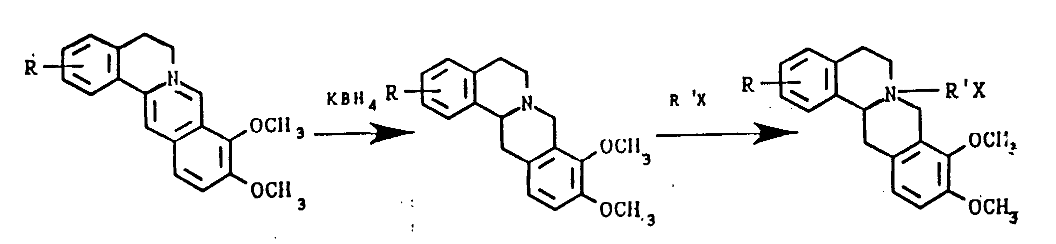
wherein R,R',X are as defined above.

Compound I13 of the invention shows marked antiarrhythmic effects upon various experimental arrhythmic models and inhibits ventricular fibrillation and myocardial ischemia. Its analogues possess the same cardiovascular activities as well.

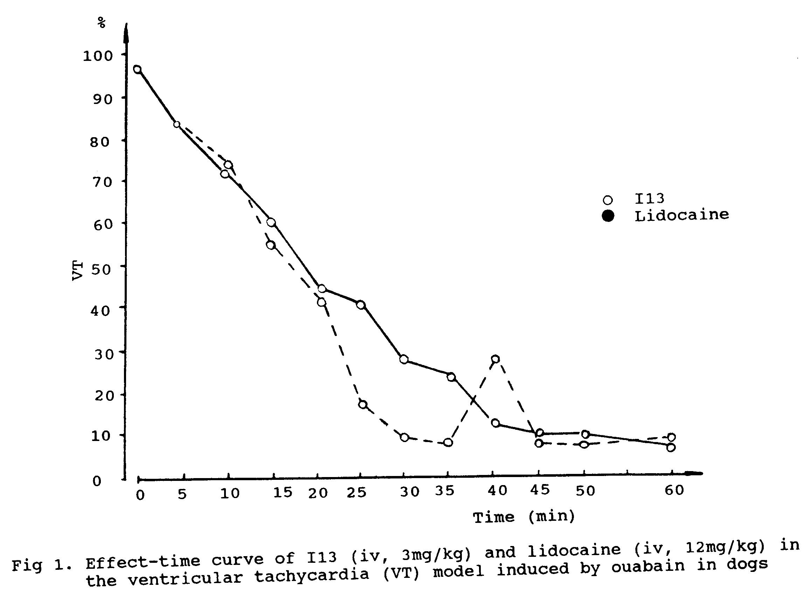

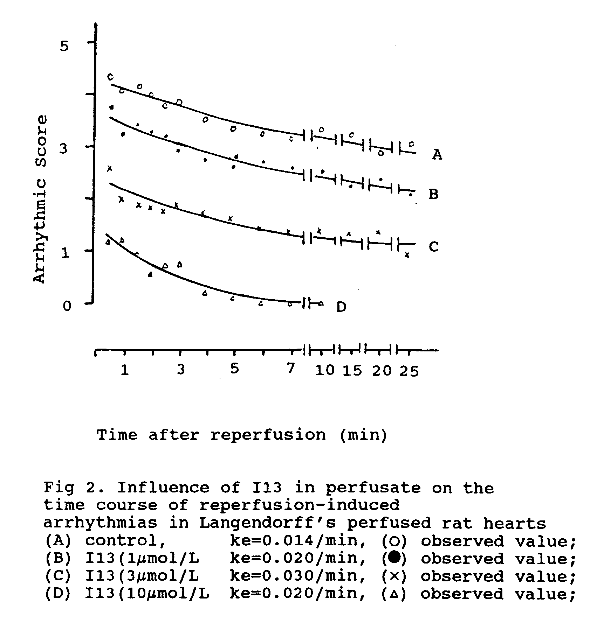

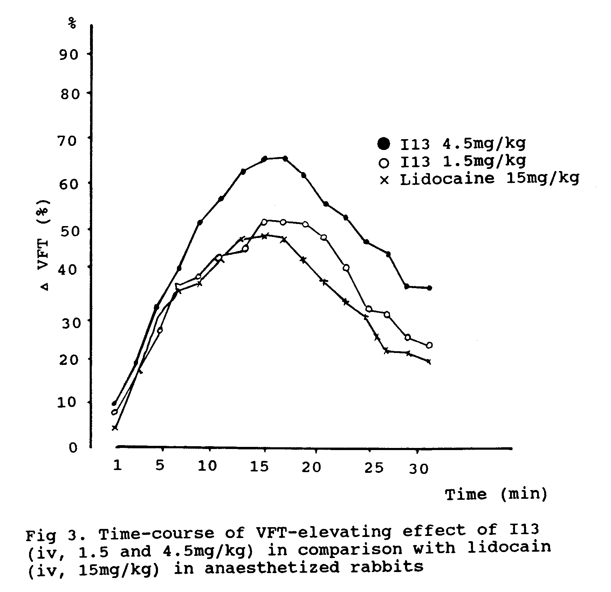

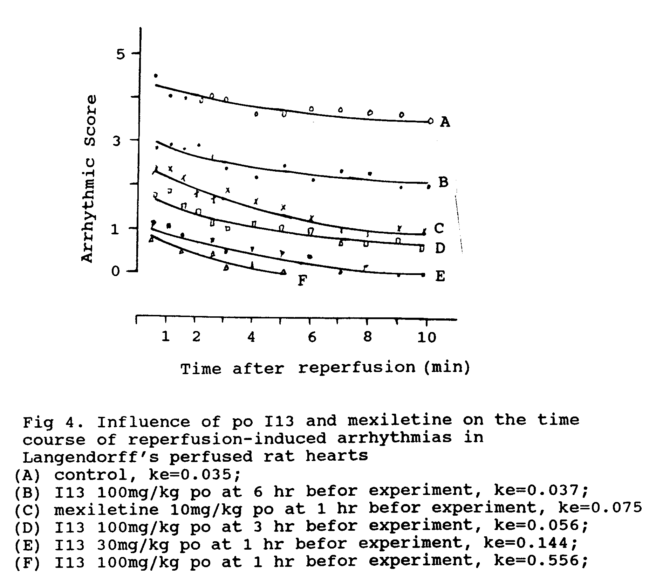

The Pharmacodynamics of I13-a Novel Antiarrhythmic Agent I13 was found to provide advanced protection against arrhythmia, ventricular fibrillation(VF) and myocardial ischemic injury, as well as other pharmacological benefits.
1. The arrhythmias and VF induced by left coronary artery ligation in anaesthetized rats were significantly inhibited by I13 administered by either i.v. or p.o. route. The ED₅₀ of I13 by i.v. route against arrhythmias and VF were 0.22 and 0.16 mg/kg, and the therapeutic indexes(TI) were 26 and 35 respectively. By p.o. route, the ED₅₀ of I13 were 16.5 and 20mg/kg, and the TI were 70 and 92 respectively. In contrast, the ED₅₀ of lidocaine given by i.v. route was 2.23mg/kg.Thus the anti-arrhythmic activity of I13 was 10 times more potent than that of lidocaine. The ED₅₀ of propafenon by p.o. route was 19.3mg/kg, according to which I13 is also considerably more potent than propafenon. (shown in Tab 1-4)
2. I13 markedly inhibited arrhythmias induced by ouabain in dogs. I13 by i.v. gtt. administration at a rate of 0.1mg/kg/min for 30 mins exerts the same antiarrhythmic effect as lidocaine at a rate of 0.4mg/kg/min, which suggests that the anti-arrhythmic activity of I13 is 4 times more potent than that of lidocaine. Moreover, the activity of I13 was more enduring reflected by the lack of reoccurrence of arrhythmia within a 4 hour period. In contrast, reoccurrences of VPBs, VT and VF were detected in lidocaine treatment. (shown in Tab 5, Fig 1)
3. I13 at the concentration of 3 µ mol/L offers effective protection against the VT and VF induced by ischemia and reperfusion on Langendorff's perfused rat hearts. (see Fig 2)
4. In the model of hypertrophic heart muscle disease caused by long term injections of L-thyroxine, after low perfusion - reperfusion or ischemia-reperfusion this induced a higher incidence of VF in hypertrophic hearts born in vitro and in vivo than in control hearts. The p.o. administration of 100mg/kg of I13 one hour prior to the ischemia course on the hypertropnic model clearly inhibited the incidence of VF and VT. (shown in Tab 6)
5. The elevation of electrically-induced ventricular fibrillation threshold (VFT) in rabbits, and the area under time-effect curve by I13 1.5mg/kg i.v. were similar to that induced by lidocaine 15mg/kg i.v., which suggests that I13 is ten times more potent than lidocaine in elevating VFT. (shown in Tab 7-9, Fig 3)
6. The Lasting-Effect of I13 Anti-arrythmic Action after p.o. Administration
   1, 3 and 6 hours after the administration of 100mg/kg of I13 by po to rats, the hearts were removed and mounted onto Langendorff's apparatus. The antiarrythmic effects were still present even after 30 minutes of washing the isolated hearts with perfusate not containing I13. This suggested that the position effects of I13 extend for a longer duration than drug concentration. (see Fig 4)
7. Electrophysiological effects of I13 on Myocardium Influence of I13 on Vₘₐₓ
   Within the range of 1-30 µmol/L of I13, the dosage dependently inhibited Vₘₐₓ from 7% to 48%. A preparation of 3µmol/L of I13 inhibited the Vₘₐₓ in the isolated papillary muscles of guinea pigs by 20%. The Vₘₐₓ of preparations under anoxia, high K⁺ and acidosis condition were also lowered by the I13. In the case of left ventricular hypertropy in rats induced by occluding the abdominal aorta, and left venticular hypertrophy the cardiomyopathy model induced by L-thryotoxin, Vₘₐₓ remained unchanged when not adminstering the drug, but the administration of I13 completely inhibited the Vₘₐₓ (shown in Tab 10-12).

### Influence of I13 on APD

At the lower concentration 0.3 µmol/L, I13 was able to lengthen APD50; Both APD50 of APD90 were prolonged at concentrations ranging between 1 and 3 µmol/L. Most notably, however, APD was shortend from 21% to 25% with the introduction of I13 at concentration ranging between 10 and 30 µmol/L.

In the instance of hypertrophic myocardium, already-lengthened APD could not be further exaggerated by I13.

I13 exhibited a two-phase effect on APD, that is, APD was lengthened at low concentrations and shortened at high concentrations. Moreover, it is important that I13 was able to prolong APD in normal papillary muscle but exerted no action on hypertrophic myocardium.

There was no influence of I13 on APA and RP.

The effecfiveness of I13 in lengthening APD is confirmed by the influence on monophasic action potential (MAP) recorded from the left ventricular activity in rabbits.

24 hours after AMI, APD is markedly shortened. This effect could be cancelled by the administration of I13 in rabbits.

The results from electrophysiological studies suggest that I13 is a novel antiarrhythmic agent, with the properties of Ia,Ib and III.

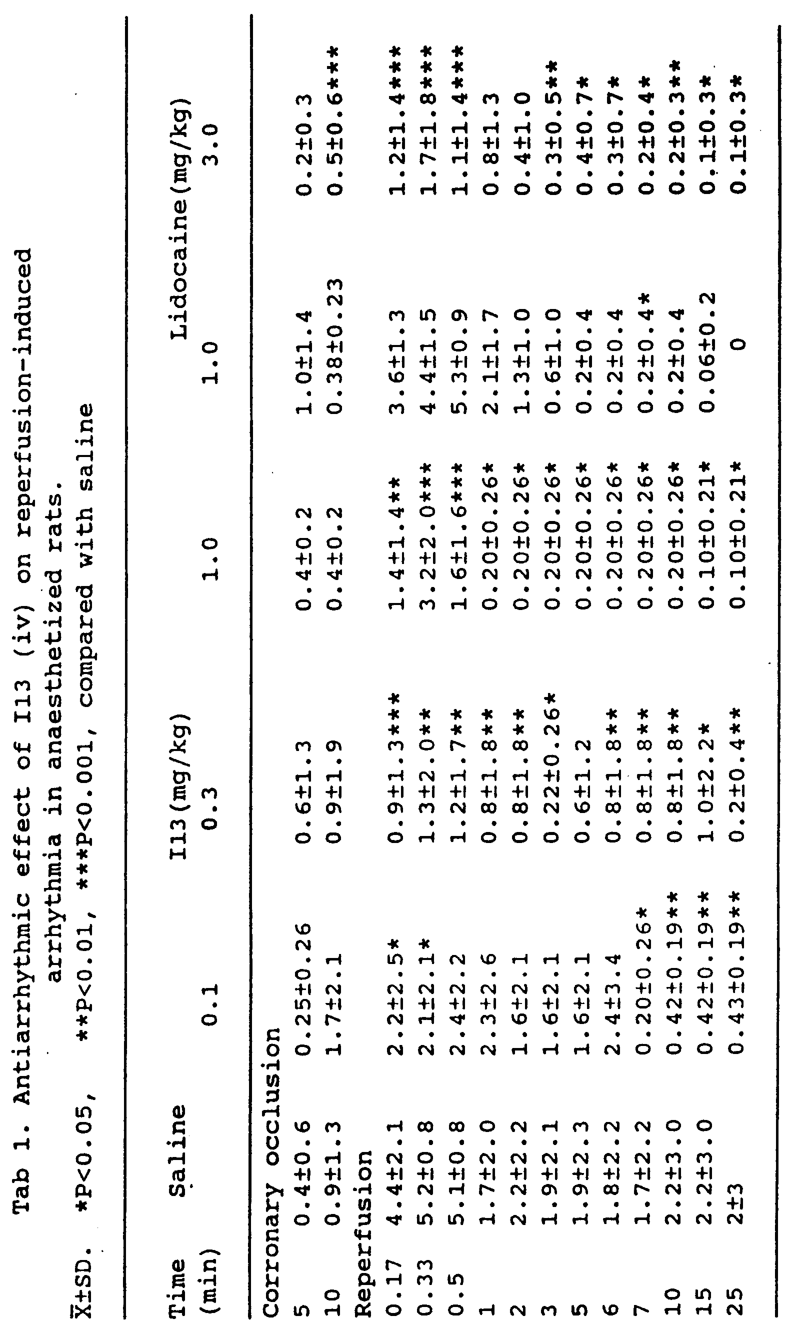

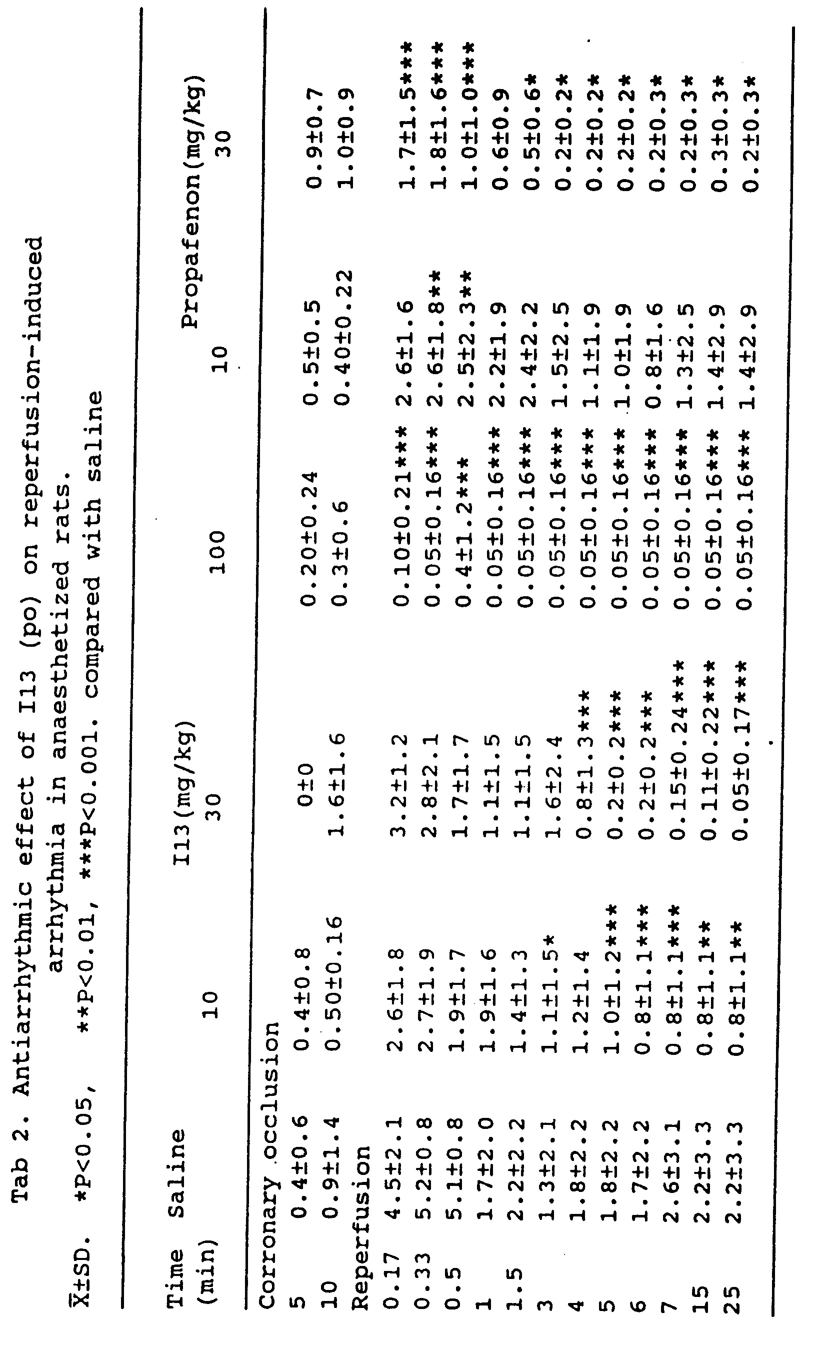

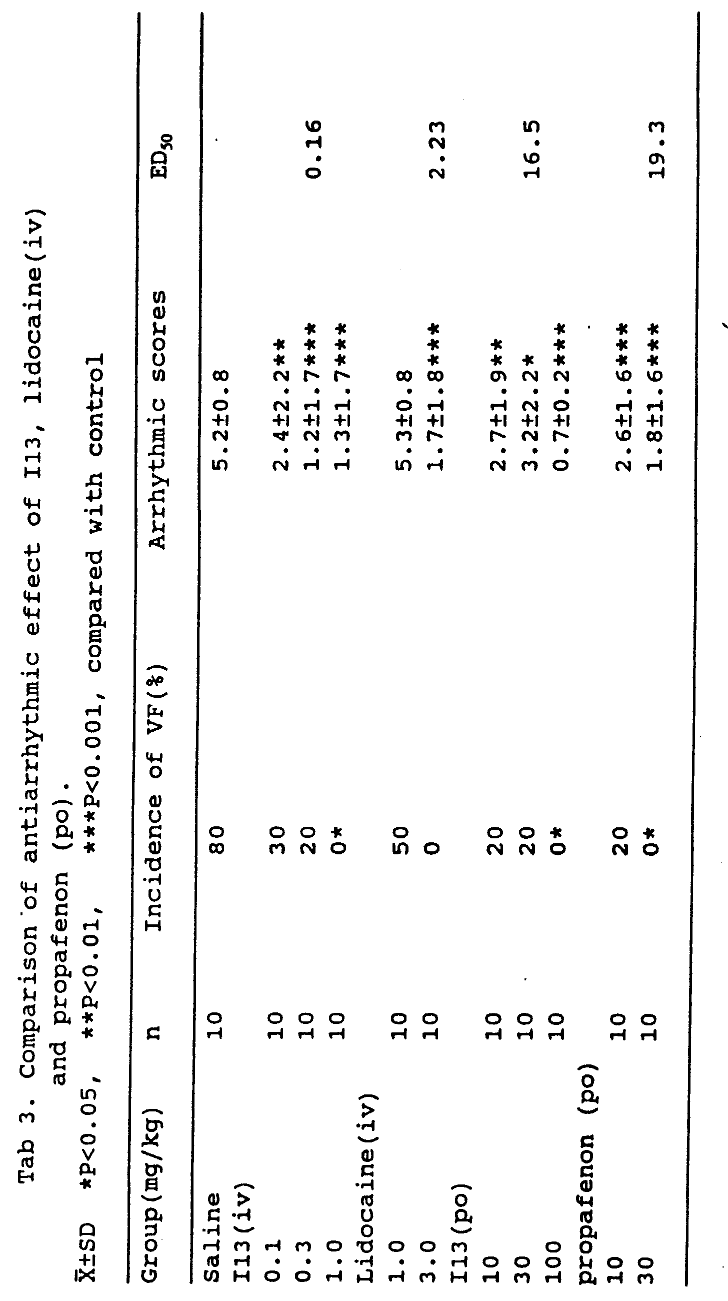

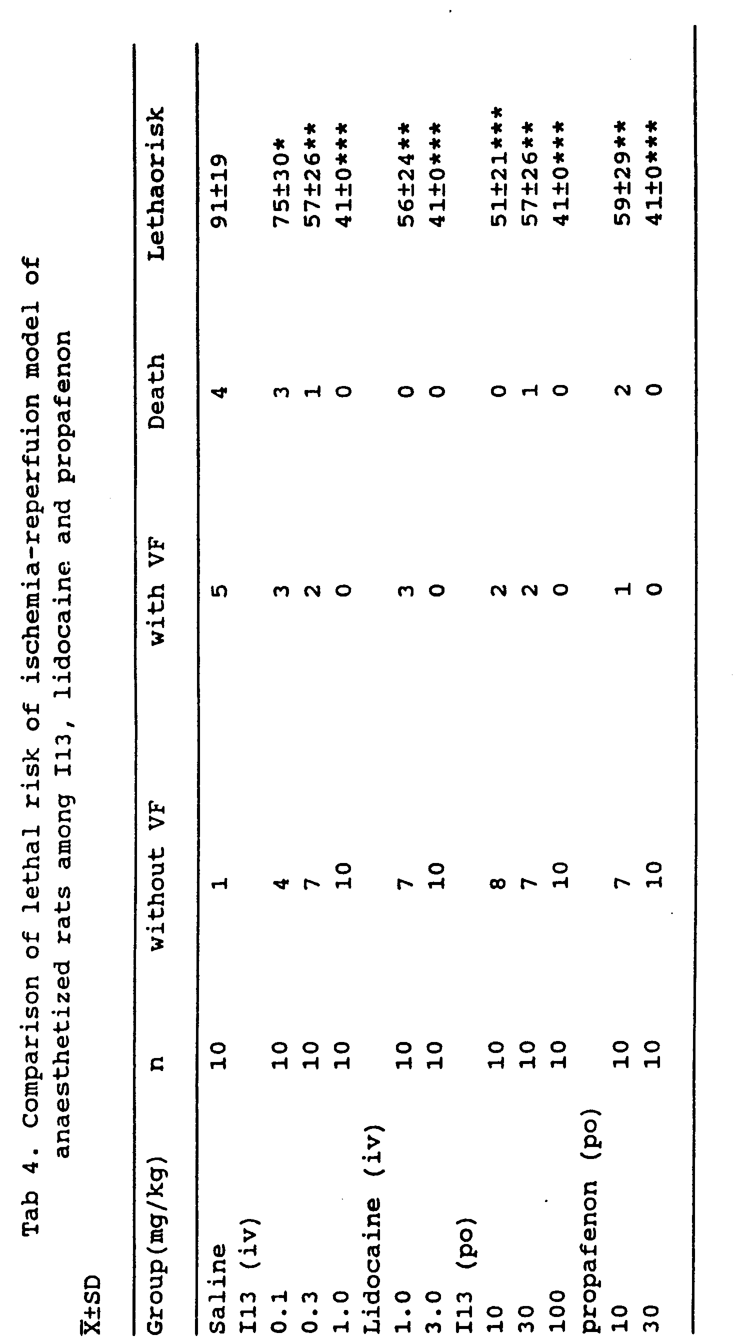

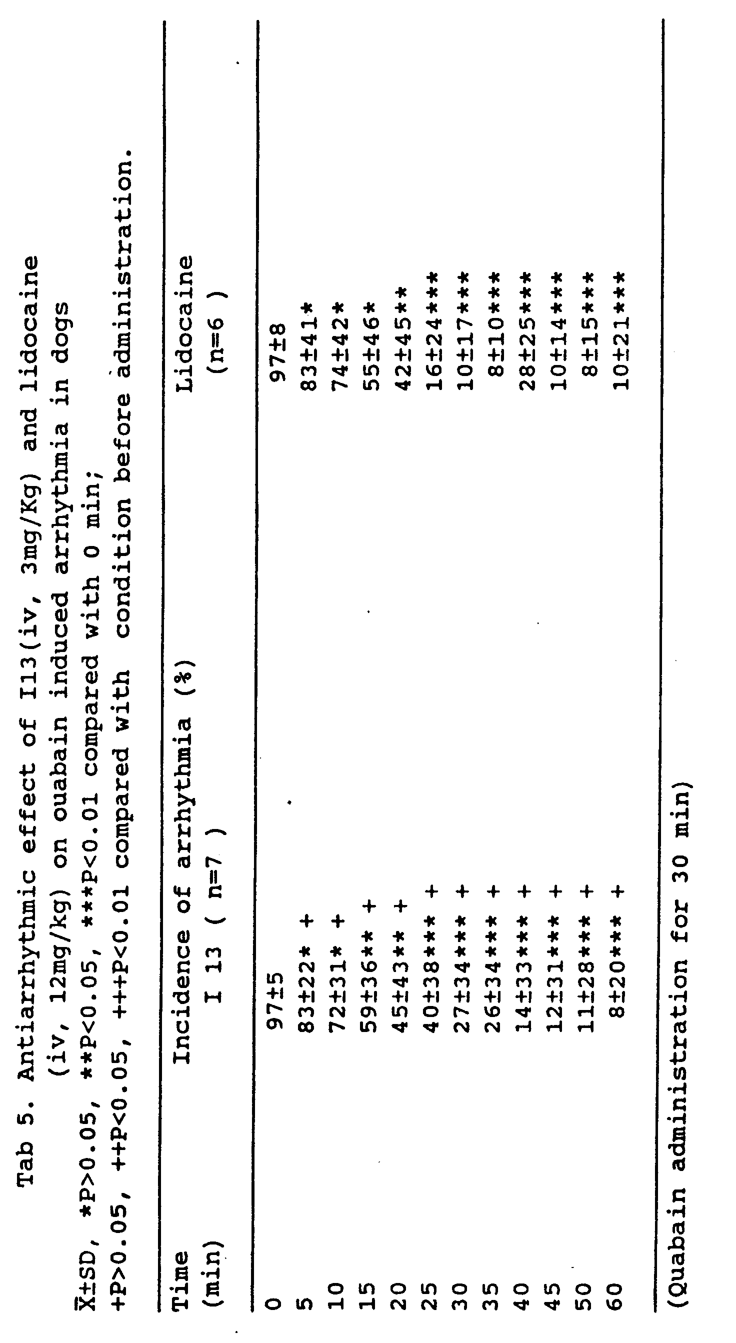

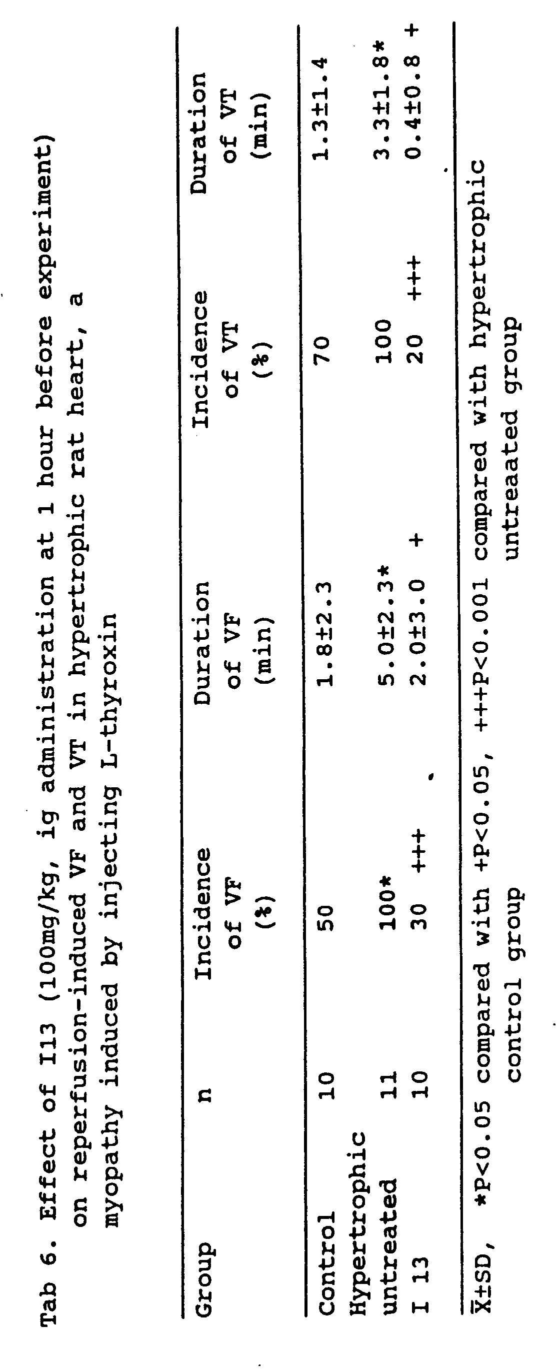

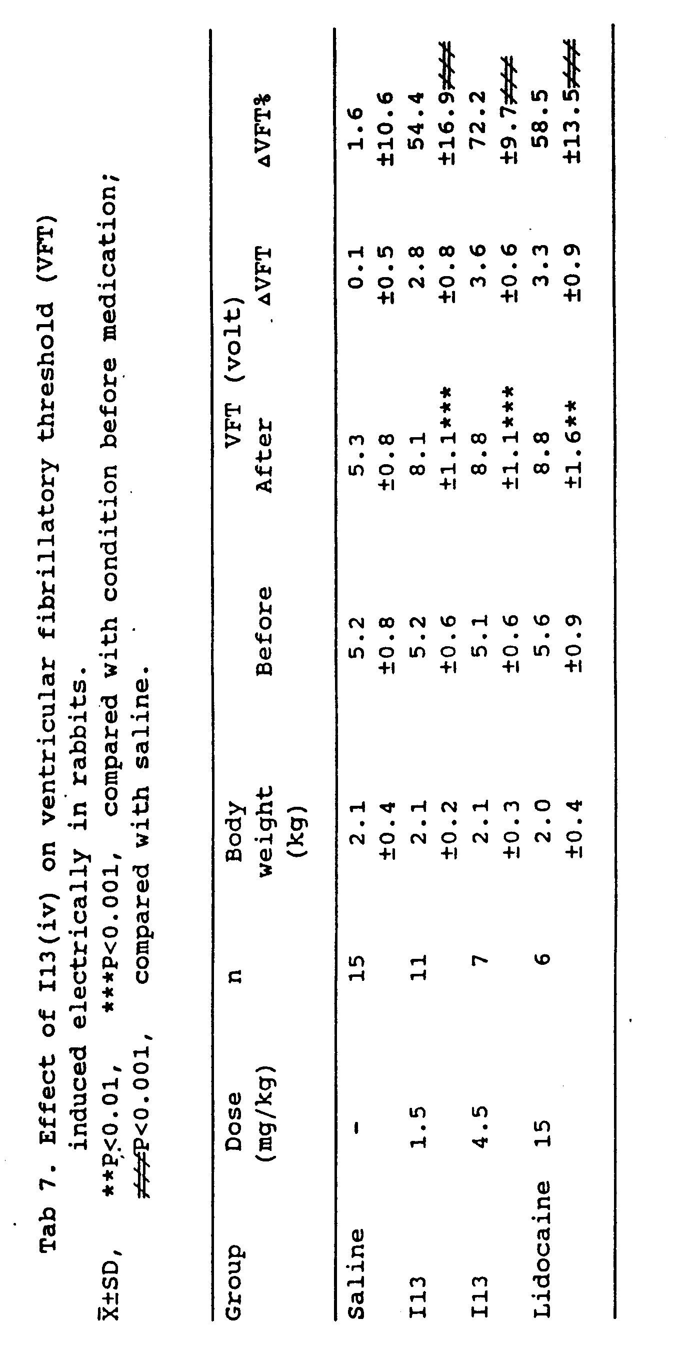

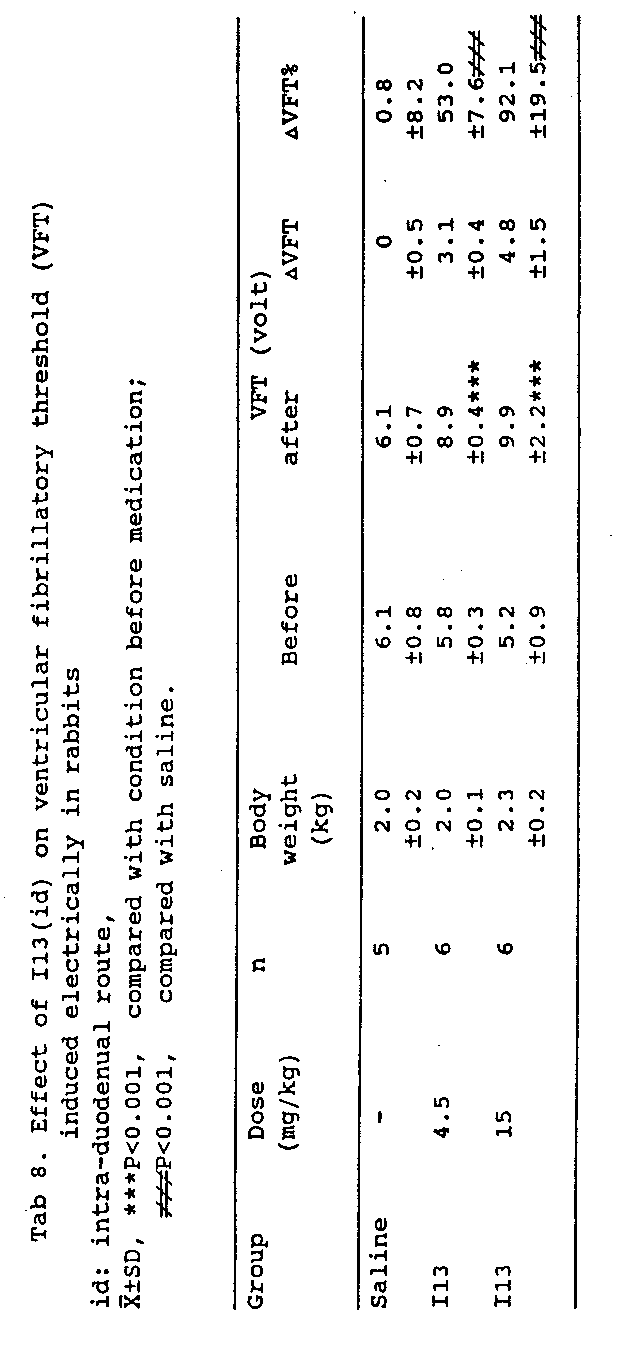

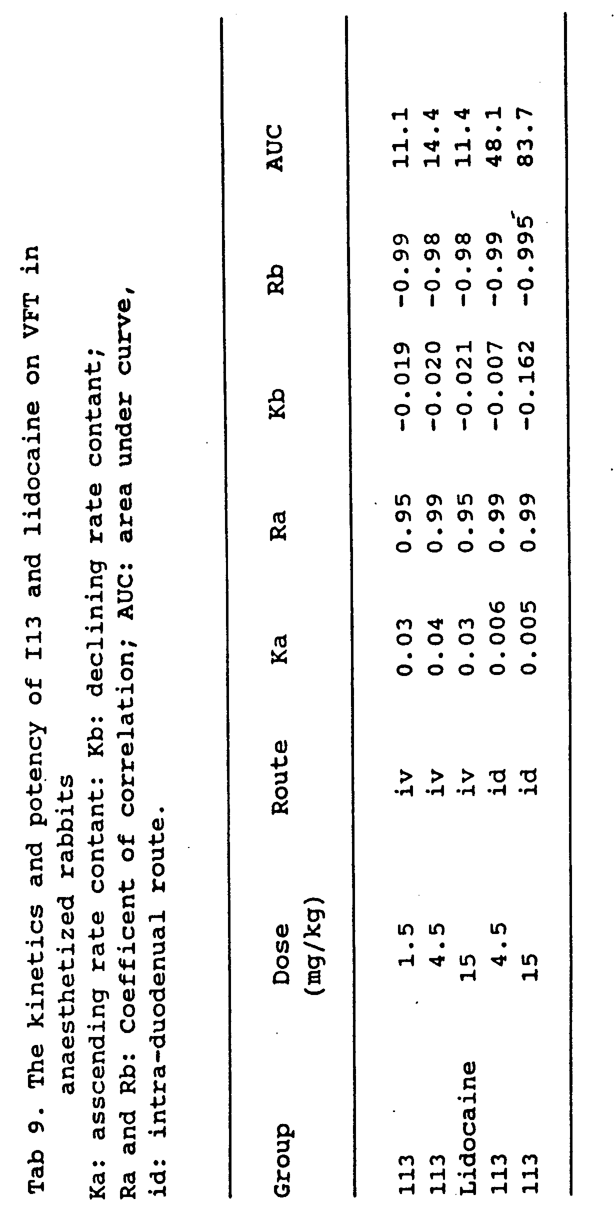

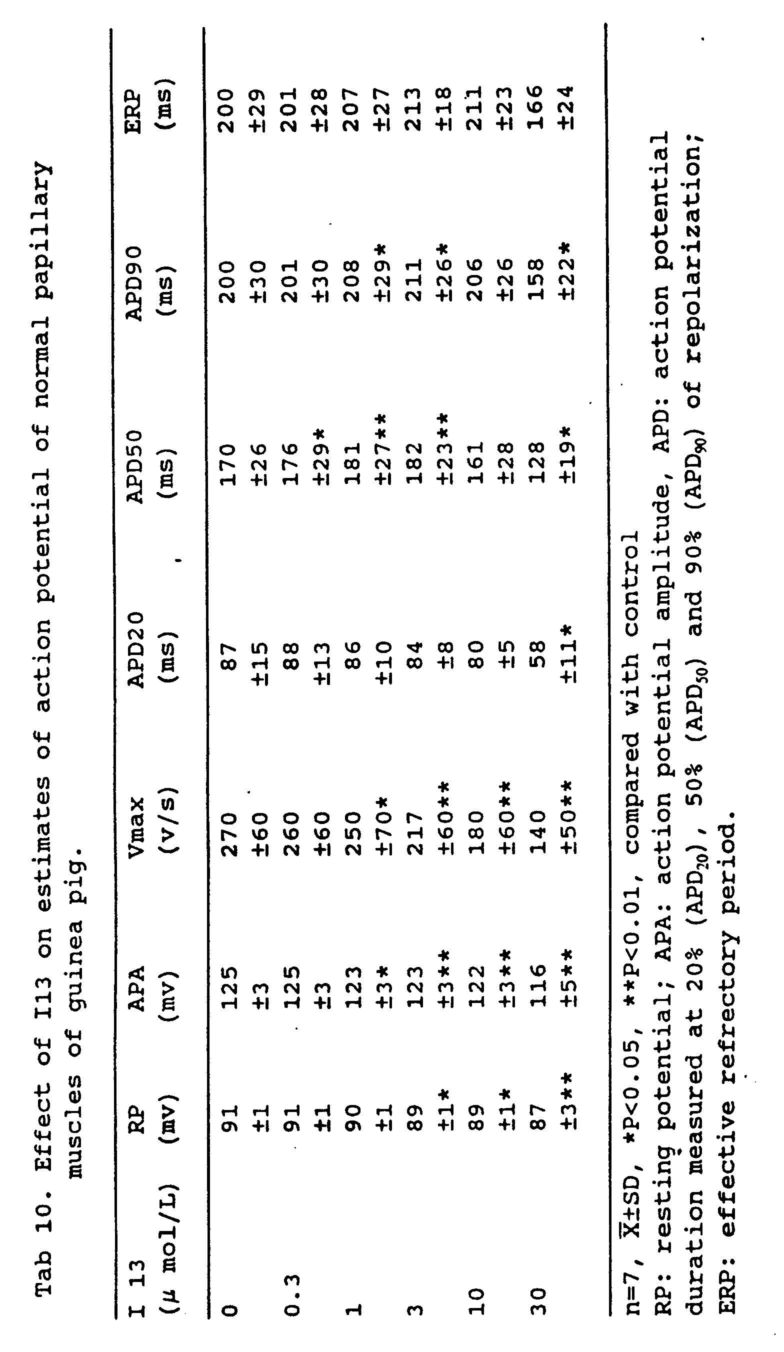

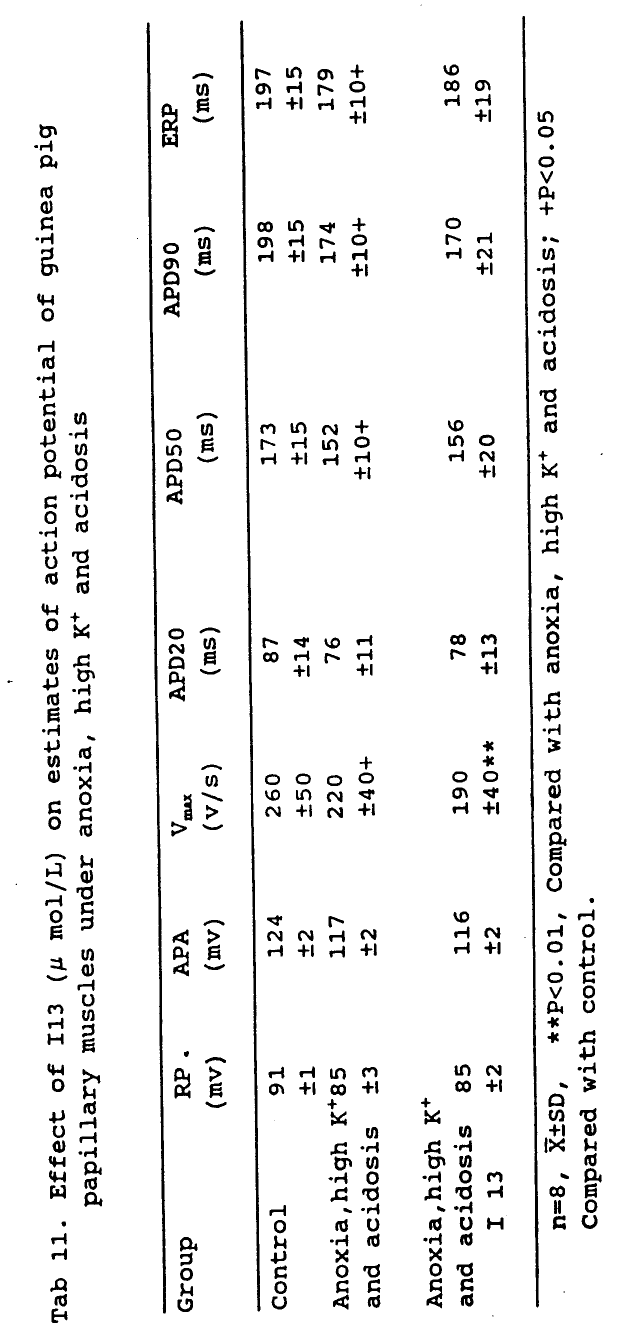

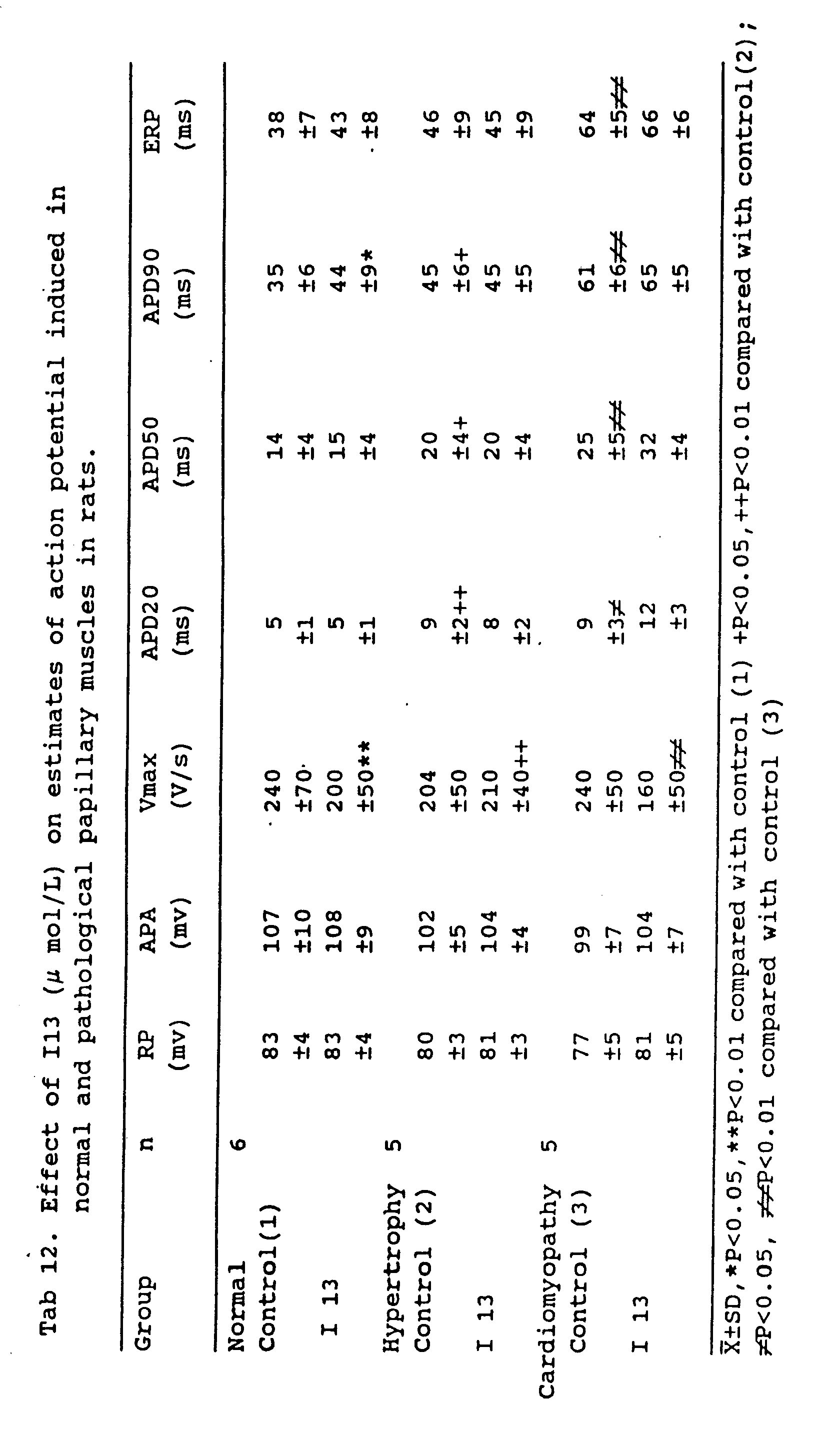

### Example 1

### 7-(4-chlorobenzyl)-5,8,13,13a-tetrahydroberberine chloride (I13)

Add 54g of tetrahydroprotoberberine(I), 33g of p-chlorobenzyl chloride and 80ml of nitromethane into a 250ml three neck bottle, heat for 6 hours under reflux. Cool, and stand in refrigerator overnight. Filter, wash with anhydrous ether to produce 63g of white powder, recrystallize with methanol to obtain 46g of white crystal (56.3%) having melting point of 208-210°C.

| Elemental analysis for C₂₇H₂₇Cl₂NO₄·1 1/2H₂O | | | |
|---|---|---|---|
| | C(%) | H(%) | N(%) |
| Calculated | 61.48 | 5.73 | 2.66 |
| Found | 61.53 | 5.56 | 2.59 |

IR ( ν$\frac{\text{KBr}}{\text{max}}$ cm⁻¹)
2907 (CH₂), 2827 (OCH₃), 1603, 1490 (Aromatic cycle), 1282, 1235,
1096, 1032 (ether bond), 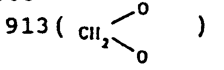
MS (m/z)
464(M⁺), 338, 308, 278, 176, 164, 149, 125
¹HNMR (δ,ppm,CD3OD)
3.86(s,3H, 10-OCH₃), 3.93(s,3H, 9-OCH₃), 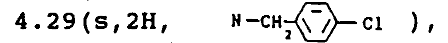
6.02(s,2H, -OCH₂O),
6.89(s,1H, 4-aromatic hydrogen), 7.45(s,1H,12-aromatic hydrogen), 7.54(s,1H, 11-aromatic hydrogen), 7.17-7.27(m,4H-, aromatic hydrogen of benzyl).

### Example 2

### 7-Benzyl-5,8,13,13a-tetrahydropalmatine chloride (II6)

Mix 3g of tetrahydropalmatine with 10ml of benzyl chloride, heat to 100-110°c for 1.5 hours. cool, add ether and stir throughly, filter and wash with ether, dry to produce 4g of white powder. Recrystallize with anhydrous ether-methanol(3:7) to obtain 3.8g of white crystal (93.4%) having a melting point of 175-176°c.

| Elemental analysis for C₂₈H₃₂CINO₄·H₂O | | | |
|---|---|---|---|
| | C(%) | H(%) | N(%) |
| Calculated | 67.26 | 6.85 | 2.80 |
| Found | 67.30 | 6.76 | 2.42 |

MS (m/z)
446 (M ), 335 (tetrahydropalmatine), 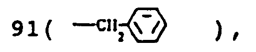 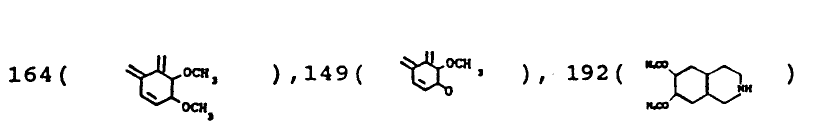
¹HNMR (δ,ppm,CD₃OD)
3.83(s,3H, 9-OCH₃), 3.55(d,1H, 8'-2H), 3.90(t,9H, 2,3,10-OCH₃),
4.25(d,1H, 8'-eH), 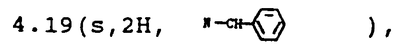
6.78-6.85 (m, 4H, 1,4,11,12-aromatic hydrogen),
7.59(s,5H, aromatic hydrogen of benzyl)
Compounds I1-9, II1-5,7-9 are prepared as compound II6.
I6
MS (m/z)
430(M⁺), 339(tetrahydropalmatine), 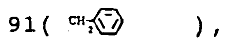 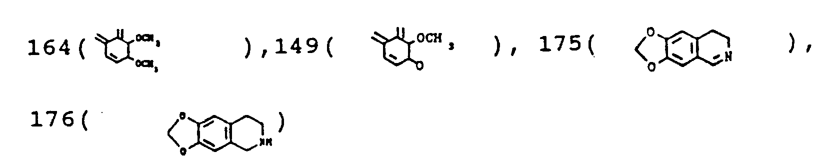
¹HNMR(δ,ppm,CD₃OD)
3.88(s, 3H, 10-OCH₃), 3.93(s, 3H, 9-OCH₃), 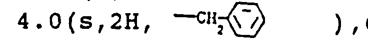
6.01(s,2H, CH₂), 6.75(s,1H, 4-aromatic hydrogen), 7.19(m,2H,11,12-aromatic hydrogen), 7.49 (s,5H, aromatic hydrogen of benzyl)

### Example 3

### 7-Benzyl-5,8,13,13a-tetrahydroberberine bromide(I10)

Add 10.2g of tetrahydroprotoberberine and 6.6g of p-nitrobenzyl bromide in 200ml of acetone, heat for 5 hours under reflux. Filter and wash with acetone to produce 12.6g of white powder. Recrystallize with methanol to obtain 11.8g of white and yellowish crystal (70.2%) having a melting point of 185-186°C.

| Elemental analysis for C₂₇H₂₇BrN₂O₆ | | | |
|---|---|---|---|
| | C(%) | H(%) | N(%) |
| Calculated | 58.48 | 4.90 | 5.04 |
| Found | 58.48 | 4.90 | 4.68 |

¹HNMR (δ,ppm,CD₃OD)
3.92(s,3H, 10-OCH₃), 3.98(s,3H, 9-OCH₃), 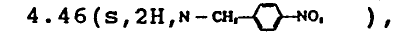
5.0[m,2H, 8-(CH₂)], 6.25(s,2H, -OCH₂O-),6.87-6.95(m,2H,1,4-aromatic hydrogen),8.1-8.5(m,4H, aromatic hydrogen of p-nitrobenzyl).

Compounds I11,II10-11 are prepared as compound I10.

### Example 4

### 7-(morpholinylethyl)-5,8,13,13a-tetrahydroberberine chloride (I12)

Add 1g of tetrahydroprotoberberine and 2g of N-chloroethylmorpholine into 25ml of ethanol, heat for 45 hours under reflux. Filter, collect filtrate and reduce to half volume under vacuum. Stand, filter to produce white powder, recrystallize with anhydrous ethanol to obtain 0.6g of white needle crystal (38.5%) having a melting point of 250-251°C.

| Elemental analysis C₂₆H₃₃ClN₂O₆ | | | |
|---|---|---|---|
| | C(%) | H(%) | N(%) |
| Calculated | 63.86 | 6.80 | 5.73 |
| Found | 64.04 | 7.13 | 5.38 |

### Example 5

### 5,8,13,13a-tetrahydroprotoberberine(I)

Add 20g of protoberberine and 500ml of 95% ethanol in a 1000ml three neck bottle, stir and heat until all protoberberine is dissolved under reflux, add 6g of potassium borohydride in small portions within 1 hour. Stop heating and stir for another 3 hours. Stand at room temperature, filter and dry to produce 14g of yellowish powder. Recrystallize with benzene to obtain 11g of white crystal (55.5%) having a melting point of 175-177°C.

## Claims

1. A tetrahydroprotoberberine quaternary ammonium compounds represented by the general formula: 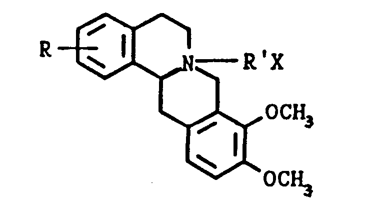 Wherein

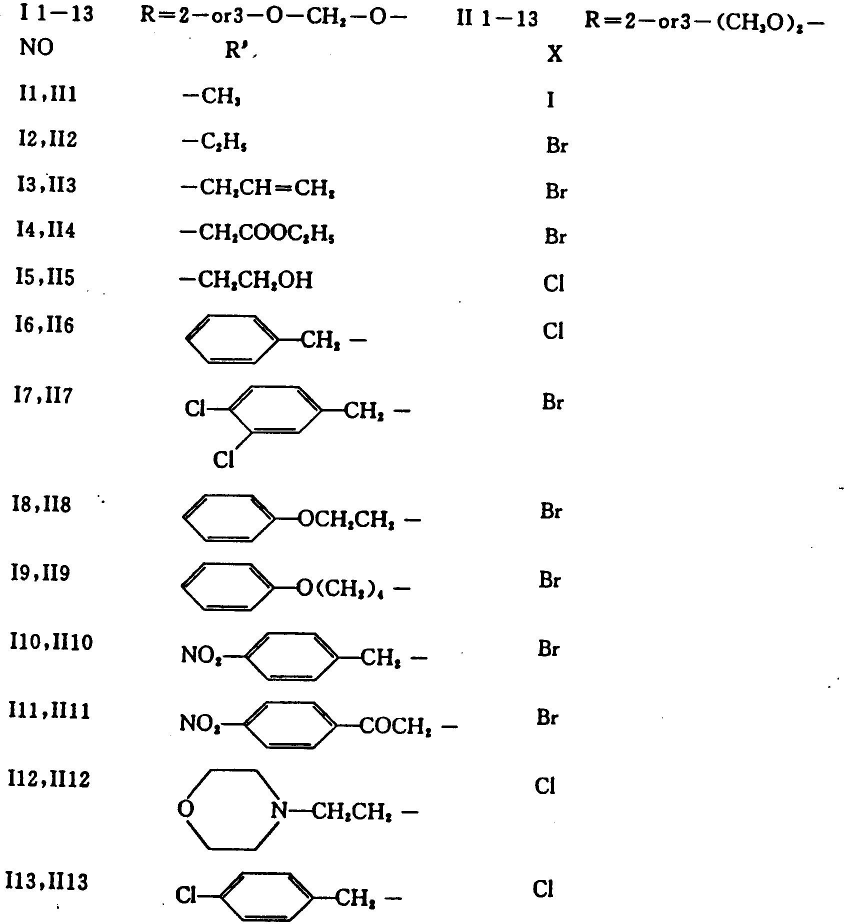

2. A process of producing a tetrahydroprotoberberine quaternary ammonium compound of the formula 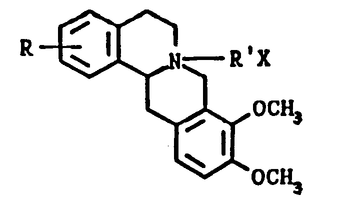 wherein 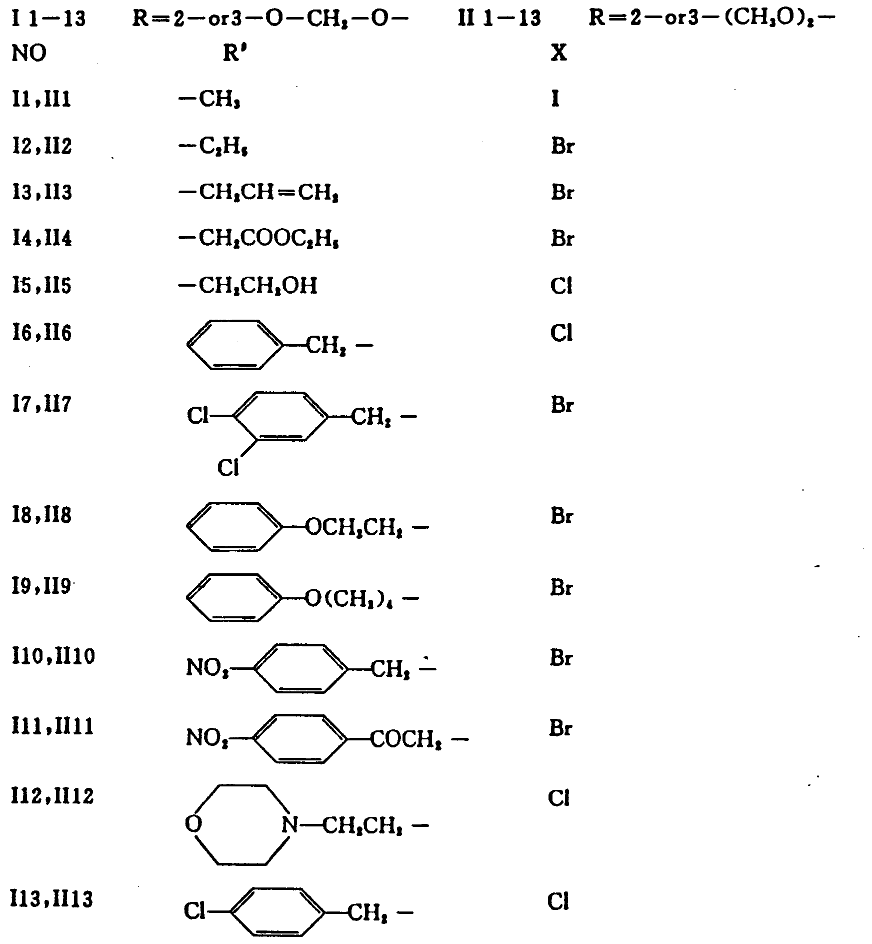 which comprises
(1) reducing protoberberine by potassium borohydride to form tetrahydroprotoberberine;and
(2) reacting tetrahydroprotoberberine with a suitable halohydrocarbon to produce tetrahydroprotoberberine quaternary ammonium compounds.

3. A process according to claim 2 which comprises using excess suitable halohydrocarbon as a solvent to react tetrahydroprotoberberine at 100-110°C for 1.5 to 10 hours.

4. A process according to claim 2 which comprises using acetone as a solvent to react tetrahydroprotoberberine with a suitable halohydrocarbon under reflux.

5. A process according to claim 2 which comprises using ethanol as a solvent to react tetrahydroprotoberberine with a suitable halohydrocarbon.
